# EUROPÄISCHE PATENTSCHRIFT

(11) **EP 1 214 002 B1**
(45) Veröffentlichungstag und Bekanntmachung des Hinweises auf die Patenterteilung: **22.10.2003**
(21) Anmeldenummer: 00974371.7
(22) Anmeldetag: 09.09.2000
(51) Int. Cl.: A61B 17/28

(54) **MEDIZINISCHES INSTRUMENT**
MEDICAL INSTRUMENT
INSTRUMENT MEDICAL

(30) Priorität: 21.09.1999 DE 19945228
(43) Veröffentlichungstag der Anmeldung: 19.06.2002
(73) Patentinhaber: Karl Storz GmbH & Co. KG, 78532 Tuttlingen (DE)
(72) Erfinder: LANG, Dieter, 96342 Stockheim (DE)
(74) Vertreter: Hofmeister, Frank Horst
(86) Internationale Anmeldenummer: EP0008814
(87) Internationale Veröffentlichungsnummer: WO01021078

(56) Entgegenhaltungen:
- DE-U- 29 820 429
- US-A- 4 646 751
- US-A- 5 290 308
- US-A- 5 489 290

## Beschreibung

Die Erfindung betrifft ein medizinisches Instrument mit einem langgestreckten Schaft und einer mit dem proximalen Ende des Schaftes verbundenen Handhabe zur Betätigung eines am distalen Ende des Schaftes angeordneten Werkzeugs, wobei der Schaft im Bereich des proximalen Endes einen verschließbaren Spülanschlußstutzen aufweist, über den, insbesondere zu Reinigungszwecken, Luft und/oder Spülflüssigkeit über einen sich an den Spülanschlußstutzen anschließenden Spülkanal hin zur Lagerung des Schaftes leitbar ist und der Spülanschlußstutzen unter einem spitzen Winkel zum Schaft ausgerichtet und zum proximalen Ende des Schaftes weisend angeordnet ist.

Bei den aus der Praxis bekannten medizinischen Instrumenten dienen der Spülanschlußstutzen und der Spülkanal dazu, den Schaft des Instruments im Bereich seiner Lagerung an der Handhabe sowie den hohlzylindrischen Teil des Schaftes mit Luft und/oder Spülflüssigkeit zu reinigen. Die Schäfte der meisten medizinischen Instrumente sind am proximalen Ende abgedichtet, um das Eindringen von Verunreinigungen zu verhindern, weshalb über den Spülanschlußstutzen eingeleitete Spülflüssigkeit nur über das distale Ende abfließen kann. Die Spülanschlußstutzen der bekannten medizinischen Instrumente sind als rechtwinklig vom Schaft fortweisende Luer-Kupplungen ausgebildet, die über separate Verschlußstopfen verschließbar sind. Neben dem Umstand, daß der Verschlußstopfen verloren gehen kann, kann die Ausbildung des Spülanschlußstutzens als rechtwinklig abstehende Luer-Kupplung nachteilig sein, da hierdurch die Sicht und/oder Bewegungsfreiheit des Operateurs eingeschränkt wird. Weiterhin nachteilig ist rechtwinklige Zuleitung der Spülflüssigkeit, da hierdurch das proximale Ende des Schaftes nicht immer ausreichend mit Spülflüssigkeit versorgt wird.

Ein gattungsgemäßes medizinisches Instrument ist aus der US 5,290,308 A bekannt. Bei diesem bekannten medizinischen Instrument ist der Spülanschlußstutzen in einem auf dem Schaft angeordneten Knauf so angeordnet, daß dieser unter einem spitzen Winkel zum Schaft ausgerichtet ist und zum proximalen Ende des Schaftes weist. Der zur Umgebung offene Spülanschlußstutzen ist an seinem unteren, im Knauf liegenden Ende durch ein federbelastetes Kugelventil verschlossen. Dieses Kugelventil verschließt den Übergang vom Spülanschlußstutzen zum eigentlichen Spülkanal. Da das Kugelventil so angeordnet ist, daß die einströmende Spülflüssigkeit zuerst die Federspannung überwinden muß, um das Ventil und somit den Zugang zum Spülkanal zu öffnen, treten beim Übergang zum Spülkanal große Druckverluste auf, so daß die Spülflüssigkeit fast drucklos in den Spülkanal eintritt, was wiederum die Reinigungskraft deutlich schwächt. Dieser Druckverlust wird bei dem bekannten Instrument noch dadurch verstärkt, daß aufgrund der Anordnung des Kugelventils die Spülflüssigkeit beim Übergang in den Spülkanal um 90° umgelenkt werden muß.

Ausgehend von diesem Stand der Technik liegt der Erfindung die **Aufgabe** zugrunde, ein medizinisches Instrument der eingangs genannten Art derart weiterzuentwickeln, daß dieses bei guter Handhabbarkeit eine sichere Reinigung über den Spülanschlußstutzen gewährleistet.

Die **Lösung** dieser Aufgabenstellung ist erfindungsgemäß dadurch gekennzeichnet, daß der Spülkanal als direkte geradlinige und freie Verlängerung des Spülanschlußstutzens ausgebildet ist und ebenfalls unter einem spitzen Winkel zum Schaft ausgerichtet zum proximalen Ende des Schaftes weisend angeordnet ist.

Durch die erfindungsgemäße Ausbildung des Spülkanals als geradlinige Verlängerung des Spülanschlußstutzens, die darüber hinaus frei von Einbauelemeten, wie beispielsweise Ventilen ist, gelangt die beispielsweise über eine Spritze in den Spülanschlußstutzen eingespritzte Spülflüssigkeit ungehindert und somit ohne Druckverlust an das proximale Ende des Schaftes. Durch diesen Druckstoß lassen sich auch festsitzende Ablagerungen zuverlässig entfernen. Der schräg zum proximalen Ende des Schaftes gerichtete Spülanschlußstutzen mitsamt Spülkanal bewirkt daher eine verbesserte Reinigung des proximalen Endes des Schaftes, da die Spülflüssigkeit direkt auf dieses Ende gerichtet eingeleitet wird.

Weiterhin wird mit der Erfindung vorgeschlagen, daß der Spülanschlußstutzen über ein am Schaft festgelegtes Verschlußelement verschließbar ist. Durch diese erfindungsgemäße Ausgestaltung ergibt sich der Vorteil, daß nicht mehr die Gefahr besteht, daß das Verschlußelement verloren gehen kann.

Gemäß einer bevorzugten Ausführungsform der Erfindung ist das Verschlußelement als koaxial auf dem Schaft angeordnete Hülse mit einem im wesentlichen radial von der Hülse fortweisenden Verschlußarm ausgebildet, wobei die Hülse um die Längsachse des Schaftes verdrehbar ist. Durch die koaxiale Anordnung der Hülse des Verschlußelements auf dem Schaft wird weiterhin sichergestellt, daß die Bauweise des medizinischen Instruments schlank und kompakt bleibt.

Um eine gute Abdichtung des Spülanschlußstutzens über das Verschlußelement sicherzustellen, wird gemäß einer praktischen Ausführungsform der Erfindung vorgeschlagen, daß das Verschlußelement in Richtung auf das proximale Ende des Schaftes und somit in Richtung auf den Spülanschlußstutzen vorgespannt ist.

Zur Erzeugung dieser Vorspannung des Verschlußelements wird vorgeschlagen, daß innerhalb der Hülse auf dem Schaft eine Druckfeder oder ein federnder Gummiring angeordnet sind. Durch diese beispielsweise koaxial auf dem Schaft angeordnete Druckfeder wird das Verschlußelement permanent in Richtung hin zum Spülanschlußstutzen gedrückt, wodurch in der Schließstellung des Verschlußelements ein sicherer Verschluß des Spülanschlußstutzens gewährleistet wird.

Die Abdichtung des Spülanschlußstutzens über den Verschlußarm kann dadurch verbessert werden, daß auf der dem Spülanschlußstutzen zugewandten Seite des Verschlußarms ein Dichtelement angeordnet ist. Das Dichtelement ist dabei vorzugsweise als in eine Bohrung im Verschlußarm einsetzbarer Stopfen ausgebildet, der bei Verschleiß leicht ausgewechselt werden kann.

Schließlich wird mit der Erfindung vorgeschlagen, daß der Schaft auswechselbar an der Handhabe festlegbar ist. Durch die Ausbildung des Spülanschlußstutzens am proximalen Ende des Schaftes ist es möglich, das medizinische Instrument sowohl mit einem fest mit der Handhabe verbundenen Schaft als auch mit einem auswechselbaren Schaft zu versehen, ohne daß hierdurch die Spülung über den Spülanschlußstutzen beeinträchtigt wird.

Weitere Merkmale und Vorteile der Erfindung ergeben sich aus der nachfolgenden Beschreibung der zugehörigen Zeichnung, in der eine Ausführungsform eines erfindungsgemäßen medizinischen Instruments beispielhaft dargestellt ist. In der Zeichnung zeigt:
- Fig. 1: eine Seitenansicht eines erfindungsgemäßen medizinischen Instruments;
- Fig. 2: eine Seitenansicht eines abnehmbaren Schaftes mit einem erfindungsgemäßen Spülanschluß;
- Fig. 3: eine teilweise geschnittene ausschnittweise Seitenansicht des medizinischen Instruments gemäß Fig. 1;
- Fig. 4: einen Längsschnitt durch das Verschlußelement des erfindungsgemäßen medizinischen Instruments und
- Fig. 5: einen Längsschnitt durch das proximale Ende des Schaftes des erfindungsgemäßen medizinischen Instrument.

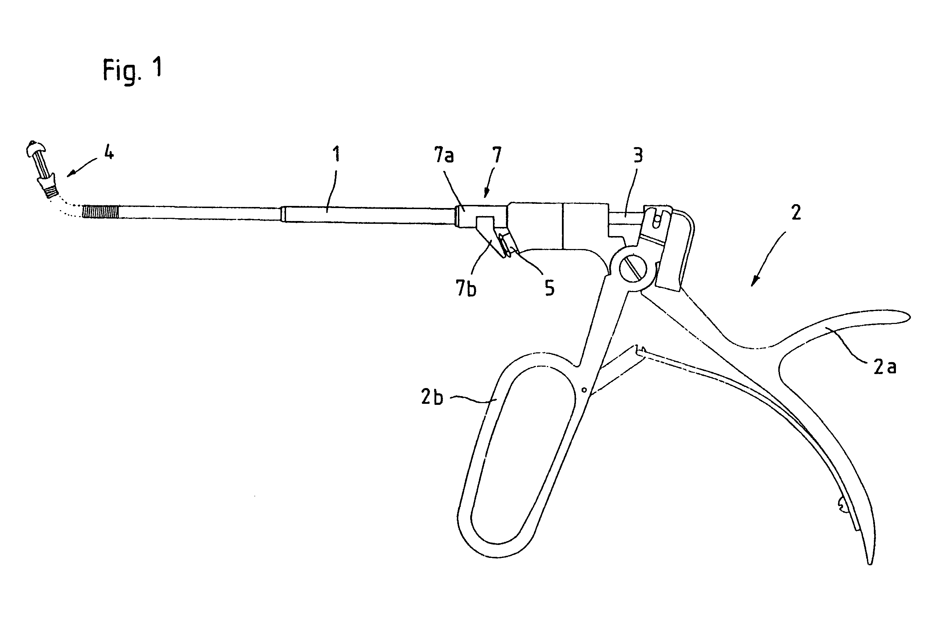

Fig. 1 zeigt ein medizinisches Instrument mit einem langgestreckten Schaft 1, und einer mit dem proximalen Ende des Schaftes 1 verbundenen Handhabe 2 mit einem starren Griffteil 2a und einem gegenüber dem starren Griffteil 2a verschwenkbaren Griffteil 2b. Über die Handhabe 2 ist mittels einer in dem Schaft 1 gelagerten Schub-/ Zugstange 3 ein Werkzeug 4 am distalen Ende des Schaftes 1 betätigbar. Bei dem dargestellten Ausführungsbeispiel handelt es sich um ein HNO-Instrument.

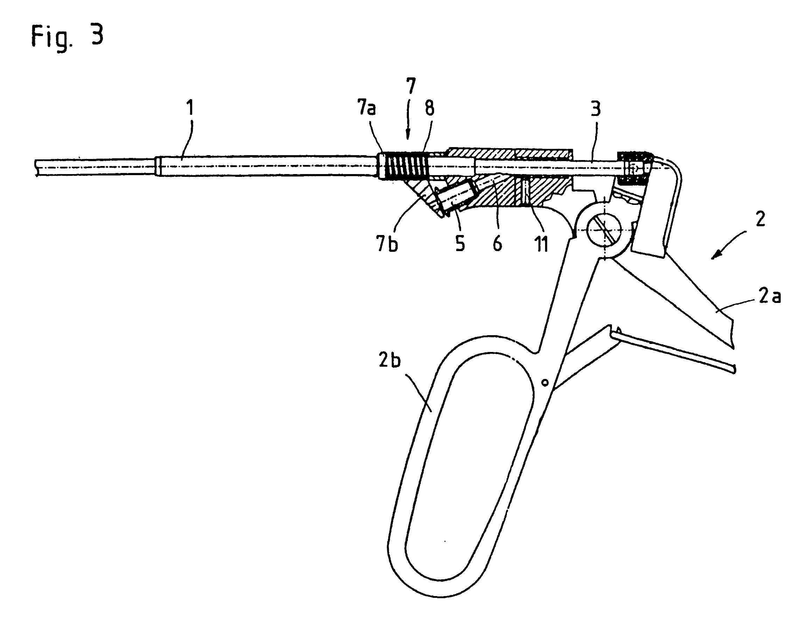

Zum Reinigen des dargestellten medizinischen Instruments und insbesondere der Lagerung des Schaftes 1 an der Handhabe 2 sowie des zur Lagerung der Schub-/Zugstange 3 dienenden hohlzylindrischen Teils des Schaftes 1 ist im Bereich des proximalen Endes des Schaftes 1 ein Spülanschlußstutzen 5 angeordnet, über den Luft und/oder Spülflüssigkeit in das medizinische Instrument eingeleitet werden kann. Über einen Spülkanal 6 gelangt die Spülflüssigkeit hin zum proximalen Ende des Schaftes 1, wie dies der Abbildung Fig. 3 zu entnehmen ist.

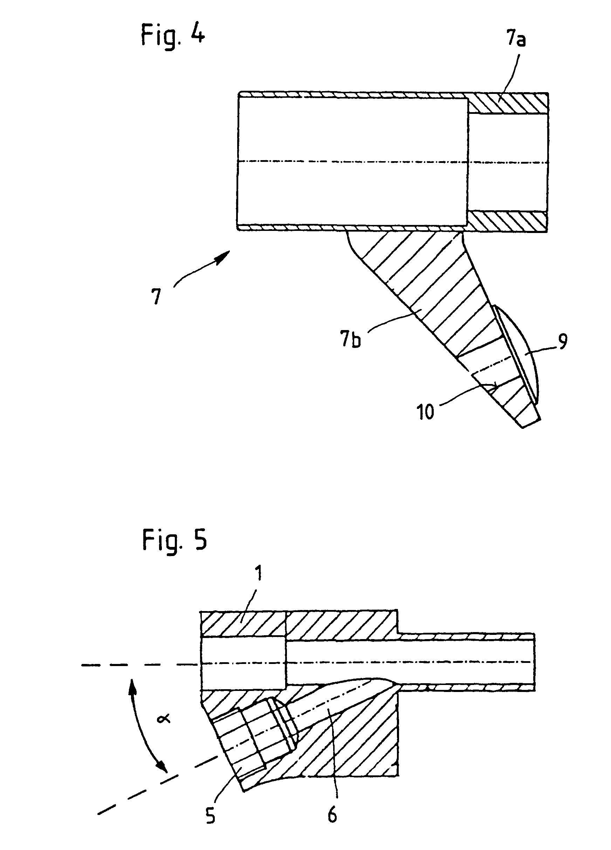

Wie aus Fig. 3 und 5 ersichtlich, sind der Spülanschlußstutzen 5 und der Spülkanal 6 unter einem spitzen Winkel α zum Schaft 1 angeordnet, um einerseits eine besonders schlanke und kompakte Bauweise des medizinischen Instruments zu ermöglichen und andererseits ein einwandfreies Reinigen des proximalen Endes des Schaftes 1 zu ermöglichen. Da das proximale Schaftende meistens abgedichtet ist, um das Eindringen von Schmutz zu vermeiden, kann die eingeleitete Spülflüssigkeit nur über das distale Ende des Schaftes 1 abfließen, weshalb es besonders vorteilhaft ist, die einzuleitende Spülflüssigkeit direkt auf das abgedichtete proximale Ende des Schaftes 1 zu lenken.

Wie weiterhin aus Fig. 1 bis 3 ersichtlich, ist der Spülanschlußstutzen 5 über ein Verschlußelement 7 verschließbar. Bei der dargestellten Ausführungsform ist besteht das Verschlußelement 7 aus einer koaxial auf dem Schaft 1 angeordneten Hülse 7a mit einem im wesentlichen radial von der Hülse 7a fortweisenden Verschlußarm 7b. Das Verschlußelement 7 ist frei um die Längsachse des Schaftes 1 verschwenkbar. Die feste Lagerung des Verschlußelements 7 auf dem Schaft 1 hat den Vorteil, daß das Verschlußelement 7 nicht verloren gehen kann.

Um das Verschlußelement 7 abdichtend gegen den Spülanschlußstutzen 5 anzudrücken, ist das Verschlußelement 7 in Richtung auf das proximale Ende des Schaftes 1 vorgespannt. Zu diesem Zweck ist bei der dargestellten Ausführungsform innerhalb der Hülse 7a eine Druckfeder 8 koaxial auf dem Schaft 1 angeordnet. Selbstverständlich ist es beispielsweise auch möglich, die Vorspannung mittels eines federnden Gummirings zu erzeugen.

In der in Fig. 1 bis 3 dargestellten Schließstellung des Verschlußelements 7 dichtet der an der Hülse 7a angeformte Verschlußarm 7b den Spülanschlußstutzen 5 ab. Diese Dichtwirkung wird dadurch verbessert, daß auf der dem Spülanschlußstutzen 5 zugewandten Seite des Verschlußarms 7b ein Dichtelement 9 angeordnet ist. Dieses im dargestellten Ausführungsbeispiel in einer Bohrung 10 des Verschlußarms 7b Dichtelement 9 soll sicherstellen, daß über den Spülanschlußstutzen 5 und den Spülkanal 6 keine Fremdstoffe in den Anschlußbereich des Schaftes 1 gelangen.

Das Betätigen des Verschlußelements 7 geschieht wie folgt:

Um ausgehend von der in Fig. 1 bis 3 dargestellten Schließstellung das medizinische Instrument reinigen zu können, muß zunächst der Spülanschlußstutzen 5 geöffnet werden. Hierzu muß die Hülse 7a gegen die Rückstellkraft der Druckfeder 8 hin zum distalen Ende des Schaftes 1 verschoben werden, um das Dichtelement 9 aus der abdichtenden Anlage am Spülanschlußstutzen 5 zu entfernen. Gleichzeitig mit dem axialen Verschieben des Verschlußelements 7 hin zum distalen Ende des Schaftes 1 kann nun die Hülse auch um die Längsachse des Schaftes 1 verdreht werden, so daß der Verschlußarm 7b nicht mehr vor dem Spülanschlußstutzen 5 liegt.

Über den Spülanschlußstutzen 5 und den Spülkanal 6 kann nun zu Reinigungszwecken Luft und/oder Spülflüssigkeit eingeleitet werden. Das Einleiten der Spülflüssigkeit kann beispielsweise über eine an den Spülanschlußstutzen 5 angesetzte Spritze erfolgen. Die Spülflüssigkeit fließt über das distale Ende des Schaftes 1 wieder ab.

Nach Beendigung des Reinigungsvorgangs wird die Hülse 7a wieder gegen die Rückstellkraft der Druckfeder 8 hin zum distalen Ende des Schaftes 1 gedrückt und gleichzeitig um die Längsachse des Schaftes 1 verdreht, bis der Verschlußarm 7b in der Schließstellung wieder das Dichtelement 9 abdichtend gegen den Spülanschlußstutzen 5 drückt.

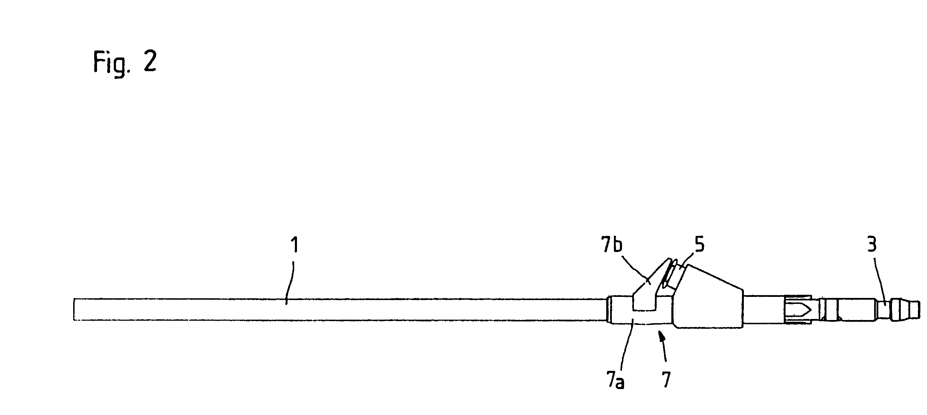

Bei dem dargestellten Ausführungsbeispiel eines medizinischen Instruments ist der Schaft 1 auswechselbar mit der Handhabe 2 verbunden, wie dies insbesondere Fig. 3 verdeutlicht. Das Festlegen des Schaftes 1 an der Handhabe 2 erfolgt über ein Sicherungselement 11, beispielsweise eine Schraube. Neben dieser Ausgestaltungsform ist es auch möglich, den Schaft 1 mit der Handhabe 2 mit einem Schnellkoppelsystem gemäß der DE 43 07 539 A1 zu verbinden, wie dies in Fig. 2 dargestellt ist.

Insgesamt gesehen zeichnet sich ein solchermaßen ausgebildetes medizinisches Instrument durch seine kompakte und schlanke Bauweise sowie die gute Reinigbarkeit aus. Die schlanke Bauweise ermöglicht dem Operateur insbesondere bei der Operation unter direkter Sicht, d.h. ohne Zuhilfenahme eines Endoskops einen guten Überblick über das Operationsgebiet bei gleichzeitig guter Bewegungsfreiheit.

### Bezugszeichenliste

- 1: Schaft
- 2: Handhabe
- 2a: starres Griffteil
- 2b: verschwenkbares Griffteil
- 3: Schub-/ Zugstange
- 4: Werkzeug
- 5: Spülanschlußstutzen
- 6: Spülkanal
- 7: Verschlußelement
- 7a: Hülse
- 7b: Verschlußarm
- 8: Druckfeder
- 9: Dichtelement
- 10: Bohrung
- 11: Sicherungselement

- α: Winkel

## Patentansprüche

1. Medizinisches Instrument mit einem langgestreckten Schaft (1) und einer mit dem proximalen Ende des Schaftes (1) verbundenen Handhabe (2) zur Betätigung eines am distalen Ende des Schaftes (1) angeordneten Werkzeugs (4), wobei der Schaft (1) im Bereich des proximalen Endes einen verschließbaren Spülanschlußstutzen (5) aufweist, über den, insbesondere zu Reinigungszwecken, Luft und/oder Spülflüssigkeit über einen sich an den Spülanschlußstutzen (5) anschließenden Spülkanal (6) hin zur Lagerung des Schaftes (1) leitbar ist und der Spülanschlußstutzen (5) unter einem spitzen Winkel (α) zum Schaft (1) ausgerichtet und zum proximalen Ende des Schaftes (1) weisend angeordnet ist,
**dadurch gekennzeichnet,**
**daß** der Spülkanal (6) als direkte geradlinige und freie Verlängerung des Spülanschlußstutzens (5) ausgebildet ist und ebenfalls unter einem spitzen Winkel (α) zum Schaft (1) ausgerichtet zum proximalen Ende des Schaftes (1) weisend angeordnet ist.

2. Medizinisches Instrument nach Anspruch 1,**daurch gekennzeichnet, daß** der Spülanschlußstutzen (5) über ein am Schaft (1) festgelegtes Verschlußelement (7) verschließbar ist.

3. Medizinisches Instrument nach Anspruch 2, **dadurch gekennzeichnet, daß** das Verschlußelement (7) als koaxial auf dem Schaft (1) angeordnete Hülse (7a) mit einem im wesentlichen radial von der Hülse (7a) fortweisenden Verschlußarm (7b) ausgebildet ist, wobei die Hülse (7a) um die Längsachse des Schaftes (1) verdrehbar ist.

4. Medizinisches Instrument nach Anspruch 2 oder 3, **dadurch gekennzeichnet, daß** das Verschlußelement (7) in Richtung auf das proximale Ende des Schaftes (1) vorgespannt ist.

5. Medizinisches Instrument nach Anspruch 4, **dadurch gekennzeichnet, daß** zur Erzeugung der Vorspannung innerhalb der Hülse (7a) eine koaxial auf dem Schaft (1) angeordnete Druckfeder (8) angeordnet ist.

6. Medizinisches Instrument nach Anspruch 4, **dadurch gekennzeichnet, daß** zur Erzeugung der Vorspannung innerhalb der Hülse (7a) auf dem Schaft (1) ein federnder Gummiring angeordnet ist.

7. Medizinisches Instrument nach mindestens einem der Ansprüche 3 bis 6 **dadurch gekennzeichnet, daß** auf der dem Spülanschlußstutzen (5) zugewandten Seite des Verschlußarms (7b) ein Dichtelement (9) angeordnet ist.

8. Medizinisches Instrument nach Anspruch 7, **dadurch gekennzeichnet, daß** das Dichtelement (9) als in eine Bohrung (10) im Verschlußarm (7b) einsetzbarer Stopfen ausgebildet ist.

9. Medizinisches Instrument nach mindestens einem der Ansprüche 1 bis 8, **dadurch gekennzeichnet, daß** der Schaft (1) auswechselbar an der Handhabe (2) festlegbar ist.

## Claims

1. Medical instrument with an elongate shaft (1) and with a handle (2) which is connected to the proximal end of the shaft (1) and is used to actuate a tool (4) arranged at the distal end of the shaft (1), said shaft (1), in the area of the proximal end, having a closable flushing connection piece (5) via which air and/or flushing liquid can be guided, particularly for cleaning purposes, via a flushing channel (6), adjacent to the flushing connection piece (5), up to the stem of the shaft (1), and the flushing connection piece (5) is oriented at an acute angle (α) with respect to the shaft (1) and is arranged facing towards the proximal end of the shaft (1), **characterized in that** the flushing channel (6) is designed as a direct, straight and free continuation of the flushing connection piece (5) and is likewise oriented at an acute angle (α) with respect to the shaft (1) and arranged facing towards the proximal end of the shaft (1).

2. Medical instrument according to Claim 1, **characterized in that** the flushing connection piece (5) can be closed by means of a closure element (7) secured on the shaft (1).

3. Medical instrument according to Claim 2, **characterized in that** the closure element (7) is designed as a sleeve (7a) arranged coaxially on the shaft (1) and with a closure arm (7b) pointing substantially radially away from the sleeve (7a), said sleeve (7a) being rotatable about the longitudinal axis of the shaft (1).

4. Medical instrument according to Claim 2 or 3, **characterized in that** the closure element (7) is pretensioned in the direction of the proximal end of the shaft (1).

5. Medical instrument according to Claim 4, **characterized in that** a pressure spring (8) is arranged coaxially on the shaft (1) inside the sleeve (7a) in order to generate the pretensioning.

6. Medical instrument according to Claim 4, **characterized in that** a resilient rubber ring (8) is arranged on the shaft (1) inside the sleeve (7a) in order to generate the pretensioning.

7. Medical instrument according to at least one of Claims 3 to 6, **characterized in that** a sealing element (9) is arranged on that side of the closure arm (7b) directed towards the flushing connection piece (5).

8. Medical instrument according to Claim 7, **characterized in that** the sealing element (9) is designed as a plug which can be inserted into a bore (10) in the closure arm (7b).

9. Medical instrument according to at least one of Claims 1 to 8, **characterized in that** the shaft (1) can be secured removably on the handle (2).

## Revendications

1. Instrument médical, comportant un tronc allongé (1) et une manette (2) reliée à l'extrémité proximale du tronc (1) et destinée à l'actionnement d'un outil (4) agencé à l'extrémité distale du tronc (1), le tronc (1) comprenant dans la zone de l'extrémité proximale un manchon de raccordement de rinçage (5) apte à être refermé via lequel, en particulier à des fins de nettoyage, de l'air et/ou du liquide de rinçage peut être amené vers la monture du tronc (1) via un canal de rinçage (6) qui se raccorde au manchon de raccordement de rinçage (5), et le manchon de raccordement de rinçage (5) est orienté sous un angle aigu (α) par rapport au tronc (1) et est dirigé vers l'extrémité proximale du tronc (1),
**caractérisé en ce que**
le canal de rinçage (6) est réalisé sous la forme d'un prolongement rectiligne libre et direct du manchon de raccordement de rinçage (5) et est également orienté sous un angle aigu (α) par rapport au tronc (1) et est dirigé vers l'extrémité proximale du tronc (1).

2. Instrument médical selon la revendication 1, **caractérisé en ce que** le manchon de raccordement de rinçage (5) peut être refermé via un élément de fermeture (7) immobilisé sur le tronc (1).

3. Instrument médical selon la revendication 2, **caractérisé en que** l'élément de fermeture (7) est réalisé sous la forme d'une douille (7a) agencée coaxialement sur le tronc (1) et comportant un bras de fermeture (7b) dirigé en éloignement sensiblement radialement de la douille (7a), la douille (7a) pouvant tourner autour de l'axe longitudinal du tronc (1).

4. Instrument médical selon l'une ou l'autre des revendications 2 et 3, **caractérisé en ce que** l'élément de fermeture (7) est précontraint en direction de l'extrémité proximale du tronc (1).

5. Instrument médical selon la revendication 4, **caractérisé en ce que** pour générer la précontrainte à l'intérieur de la douille (7a), un ressort de compression (8) est agencé coaxialement sur le tronc (1).

6. Instrument médical selon la revendication 4, **caractérisé en ce que** pour générer la précontrainte à l'intérieur de la douille (7a), un anneau élastique en caoutchouc est agencé sur le tronc (1).

7. Instrument médical selon l'une des revendications 3 à 6, **caractérisé en ce qu'**un élément d'étanchéité (9) est agencé sur le côté du bras de fermeture (7b) tourné vers le manchon de raccordement de rinçage (5).

8. Instrument médical selon la revendication 7, **caractérisé en ce que** l'élément d'étanchéité (9) est réalisé sous la forme d'un bouchon susceptible d'être mis en place dans un perçage (10) du bras de fermeture (7b).

9. Instrument médical selon l'une des revendications 1 à 8, **caractérisé en ce que** le tronc (1) est susceptible d'être immobilisé de façon interchangeable sur la manette (2).
